# EUROPÄISCHE PATENTSCHRIFT

(11) **EP 2 144 856 B1**
(45) Veröffentlichungstag und Bekanntmachung des Hinweises auf die Patenterteilung: **14.08.2013**
(21) Anmeldenummer: 08749726.9
(22) Anmeldetag: 25.04.2008
(51) Int. Cl.: C04B 35/119, A61L 27/10, C04B 35/117, C04B 35/105, C04B 35/106

(54) **VERWENDUNG EINES SINTERFORMKÖRPERS IN DER MEDIZINTECHNIK**
USE OF A SINTERED MOULDED BODY IN MEDICAL TECHNOLOGY
UTILISATION D' UN CORPS MOULÉ FRITTÉ DANS LA TECHNIQUE MEDICALE

(30) Priorität: 27.04.2007 DE 102007020471
(43) Veröffentlichungstag der Anmeldung: 20.01.2010
(73) Patentinhaber: CeramTec GmbH, 73207 Plochingen (DE)
(72) Erfinder: KUNTZ, Meinhard, 73733 Esslingen (DE); HERRÁN FUERTES, Ana, 70180 Stuttgart (DE); FRIEDERICH, Kilian, 73207 Plochingen (DE); SCHNEIDER, Norbert, 73614 Schorndorf (DE)
(74) Vertreter: Uppena, Franz
(86) Internationale Anmeldenummer: PCT/EP2008/055059
(87) Internationale Veröffentlichungsnummer: WO 2008/132159

(56) Entgegenhaltungen:
- EP-A- 1 188 729
- WO-A-92/02470
- DE-A1- 4 116 008
- DE-A1- 19 850 366
- US-A- 5 002 911
- BURGER W: "Umwandlungs- und plateletverstärkte Aluminiumoxidmatrixwerkstoffe (Teil 2)" KERAMISCHE ZEITSCHRIFT, DVS VERLAG, DUESSELDORF, DE, Bd. 50, Nr. 1, 1. Januar 1998 (1998-01-01), Seiten 18,20-22, XP002098039 ISSN: 0023-0561
- BURGER W: "Umwandlungs- und plateletverstärkte Aluminiumoxidmatrixwerkstoffe (Teil 1)" KERAMISCHE ZEITSCHRIFT, DVS VERLAG, DUESSELDORF, DE, Bd. 49, Nr. 12, 1. Dezember 1997 (1997-12-01), Seiten 1067-1070, XP002098038 ISSN: 0023-0561

## Beschreibung

Die Erfindung betrifft die Verwendung eines Sinterformkörpers gemäß Anspruch 1.

Sinterformkörper bieten eine Vielzahl von Anwendungsmöglichkeiten. Ihre Zusammensetzung kann durch gezielte Zugabe von bestimmten Elementen und/oder deren Verbindungen auf den jeweils vorgesehenen Einsatz abgestimmt werden. Aluminiumoxid und Zirkonoxid beispielsweise sind keramische Werkstoffe, die einzeln oder in Kombination miteinander unter anderem zu Sinterformkörpern wie Schneidwerkzeugen, Katalysatorträger oder Prothesen verarbeitet werden.

Aufgabe der Erfindung ist es, einen Sinterformkörper aus einem keramischen Werkstoff zu verwenden, der optimale Eigenschaften wie Härte, Elastizität und Wärmeleitfähigkeit in sich vereint und insbesondere für medizintechnische Anwendungen geeignet ist.

In überraschender Weise hat sich gezeigt, dass sich ein Sinterformkörper der folgenden Zusammensetzung insbesondere für die Anwendung im medizintechnischen Bereich eignet, beispielsweise für die Anwendung als Orthese oder Endoprothese, wie Hüftgelenk- und Kniegelenkimplantate.

| **Wekstoffzusammensetzung** | **Formel** | **Volumenanteil** |
|---|---|---|
| Aluminiumoxid mit Chromdotierung | Al₂O₃:Cr | 70 % - 90 % |
| Zirkonoxid mit Y-Stabilisierung | ZrO₂:Y | 12 % - 22 % |
| Strontiumaluminat (mit variabler Cr-Dotierung) | SrAl₁₂₋ₓCr_{X}O₁₉ | 1 % - 5 % |

Dominierender Gefügebestandteil eines solchen Sinterformkörpers ist das Aluminiumoxid. Daher liegen die eigenschaftsbestimmenden Merkmale wie Härte, Elastizitäts-Modul und Wärmeleitfähigkeit dicht an den Eigenschaften von reinem Aluminiumoxid. Die Bestandteile Zirkonoxid und Strontiumaluminat sind in der Aluminiumoxidmatrix eingelagert. Das Strontiumaluminat bildet charakteristische plättchenförmige Kristallite, Platelets, die wesentlich zur Festigkeitssteigerung beitragen.

Die Bestandteile Zirkonoxid und Strontiumaluminat tragen zur Steigerung der Risszähigkeit bei, die um etwa 60% höher liegt als bei reinem Aluminiumoxid. Durch diese Verstärkungskomponenten wird die Festigkeit fast um den Faktor 2 gesteigert, gleichzeitig steigt die Schadenstoleranz, das heißt die Eigenschaft des Sinterformkörpers, auch bei einer möglichen Beschädigung noch eine hohe Restfestigkeit zu behalten.

Bei hoher mechanischer Belastung des erfindungsgemäßen Sinterkörpers werden überraschenderweise Mechanismen aktiviert, die beispielsweise eine Rissausbreitung hemmen oder stoppen. Der wichtigste Mechanismus ist dabei die spannungsinduzierte Umwandlung des Zirkonoxids von der tetragonalen zur monoklinen Phase. Die mit der Umwandlung einhergehende Volumenvergrößerung des Zirkonoxids bewirkt die Ausbildung lokaler Druckspannungen, die der äußeren Zugbelastung entgegenwirkt und somit das Risswachstum behindert.

Durch die eingelagerten Platelets wird überraschenderweise der Risspfad abgelenkt, so dass zusätzliche Energie bei der Rissausbreitung absorbiert wird.

Als Besonderheit des Sinterformkörpers ist anzusehen, dass sich die beiden Mechanismen wechselseitig verstärken, so dass die effektive Steigerung der Risszähigkeit sogar größer ist, als es durch simple Addition der Einzelmechanismen zu erwarten wäre.

Die Herstellung von Sinterformkörpern erfolgt mittels konventioneller Keramiktechnologie. Die wesentlichen Prozessschritte sind:
a) Pulvermischung gemäß vorgegebener Zusammensetzung in Wasser ansetzen, Verwendung von Verflüssigern zur Vermeidung der Sedimentation.
b) Homogenisieren im Dissolver (schnelllaufender Rührer).
c) Mahlen in Rührwerkskugelmühle, dabei Erhöhung der spezifischen Oberfläche der Pulvermischung (= Zerkleinerung).
d) Zugabe von organischen Bindern.
e) Sprühtrocknen, dabei entsteht ein rieselfähiges Granulat mit definierten Eigenschaften.
f) Befeuchten des Granulats mit Wasser.
g) Axial oder isostatisch pressen.
h) Spanabhebende Grünbearbeitung, dabei wird unter Berücksichtigung der Sinterschwindung weitgehend die Endkontur abgebildet.
i) Vorbrand, dabei Schwindung auf ca. 98% der theoretischen Dichte. Die noch verbleibenden Restporen sind nach außen geschlossen.
j) Heißisostatisches Pressen unter hoher Temperatur und hohem Gasdruck, dadurch praktisch vollständige Endverdichtung.
k) Sogenannter Weißbrand, dadurch wird das beim heißisostatischen Pressen erzeugte Ungleichgewicht der Sauerstoffionen in der Keramik ausgeglichen.
l) Hartbearbeitung durch Schleifen und Polieren.
m) Tempern.

Die Eigenschaften des Sinterformkörpers können noch durch Einlagerungen verstärkt werden. So ist es möglich, Whisker und/oder Fasern vor der Ausformung eines Sinterkörpers in den Werkstoff zu mischen oder netzartige Strukturen oder Gewebe in den Werkstoff im Grünzustand einzuarbeiten. Die Whisker, Fasern oder Netze oder Gewebe müssen aus einem Werkstoff sein, der nicht mit dem keramischen Werkstoff in der Weise in Wechselwirkung tritt, dass eine Verschlechterung seiner Eigenschaften eintritt. Außerdem darf sich der Werkstoff während des Sinterns nicht in einer Weise verändern, dass der Werkstoff geschädigt wird.

Die Sinterformkörper vereinen überraschenderweise die jeweils besten Eigenschaften von Sinterformkörpern aus reinem Aluminiumoxid und Zirkonoxid für Implantatanwendungen in sich: Härte, Alterungsbeständigkeit, Benetzungsverhalten gegenüber Wasser und hohe Wärmeleitfähigkeit sind Eigenschaften, die von Sinterformkörpern aus Aluminiumoxid bekannt sind, hohe Festigkeit und hohe Risszähigkeit, das heißt Schadenstoleranz, sind Eigenschaften, die von Sinterformkörpern aus Zirkonoxid bekannt sind.

## Patentansprüche

1. Verwendung eines Sinterformkörpers in der Medizintechnik, wobei der Sinterformkörper 70 bis 90 Volumenanteile Aluminiumoxid mit Chromdotierung (Al₂O₃:Cr), 12 bis 22 Volumenanteile Zirkonoxid mit Y-Stabilisierung (ZrO₂:Y) und 1 bis 5 Volumenanteile Strontiumaluminat der Formel SrAl₁₂₋ₓCrₓO₁₉ mit variabler Cr-Dotierung enthält.

2. Verwendung nach Anspruch 1, **dadurch gekennzeichnet, dass** die Bestandteile Zirkonoxid und Strontiumaluminat in der Aluminiumoxidmatrix eingelagert sind.

3. Verwendung nach Anspruch 1 oder 2, **dadurch gekennzeichnet, dass** das Strontiumaluminat in Form von plättchenförmigen Kristalliten, Platelets, vorliegt.

4. Verwendung nach einem der Ansprüche 1 bis 3, **dadurch gekennzeichnet, dass** der Werkstoff zusätzlich mit Whiskern und/oder Fasern oder netzartigen Strukturen oder Geweben aus geeigneten Werkstoffen durchsetzt ist.

5. Verwendung des Sinterformkörpers gemäß den Ansprüchen 1 bis 4 als Orthese und Endoprothese.

6. Verwendung des Sinterformkörpers gemäß den Ansprüchen 1 bis 4 als Hüftgelenk- oder Kniegelenkimplantat.

## Claims

1. Use of a sintered moulded body in medical technology, wherein the sintered moulded body contains 70 to 90 parts by volume aluminium oxide with chromium doping (Al₂O₃:Cr), 12 to 22 parts by volume zirconium oxide with Y stabilisation (ZrO₂:Y) and 1 to 5 parts by volume strontium aluminate with the formula SrAl₁₂₋ₓCrₓO₁₉ with variable Cr doping.

2. Use according to claim 1, **characterised in that** the components zirconium oxide and strontium aluminate are embedded in the aluminium oxide matrix.

3. Use according to claim 1 or 2, **characterised in that** the strontium aluminate is present in the form of plate-like crystallites, platelets.

4. Use according to one of claims 1 to 3, **characterised in that** the material is additionally interspersed with whiskers and/or fibres or net-like structures or woven fabrics made of suitable materials.

5. Use of the sintered moulded body according to claims 1 to 4 as an orthosis and endoprosthesis.

6. Use of the sintered moulded body according to claims 1 to 4 as a hip-joint or knee-joint implant.

## Revendications

1. Utilisation, dans une technique médicale, d'un corps façonné fritté qui contient de 70 à 90 parties en volume d'oxyde d'aluminium dopé au chrome (Al₂O₃:Cr), de 12 à 22 parties en volume d'oxyde de zirconium stabilisé à l'yttrium (ZrO₂:Y), et de 1 à 5 parties en volume d'aluminate de strontium dopé avec une quantité variable de chrome, de formule SrAl₁₂₋ₓCrₓO₁₉.

2. Utilisation conforme à la revendication 1, **caractérisée en ce que** les constituants oxyde de zirconium et aluminate de strontium sont incorporés en couches au sein de la matrice d'oxyde d'aluminium.

3. Utilisation conforme à la revendication 1 ou 2, **caractérisée en ce que** l'aluminate de strontium se présente sous forme de cristallites de forme plate appelés « plaquettes ».

4. Utilisation conforme à l'une des revendications 1 à 3, **caractérisée en ce que** le matériau est en outre entremêlé de trichites et/ou de fibres ou de structures réticulées ou d'étoffes en matériaux appropriés.

5. Utilisation d'un corps façonné fritté, conforme à l'une des revendications 1 à 4, en tant qu'orthèse ou endoprothèse.

6. Utilisation d'un corps façonné fritté, conforme à l'une des revendications 1 à 4, en tant qu'implant d'articulation de la hanche ou du genou.
